Europäisches Patentamt

European Patent Office

Office européen des brevets

(19)

(11) Publication number: **0 121 012**
**B1**

(12) **EUROPEAN PATENT SPECIFICATION**

(45) Date of publication of patent specification: **13.05.87**

(51) Int. Cl.⁴: **A 61 K 7/18, A 61 K 33/30**

(21) Application number: **83301832.8**

(22) Date of filing: **31.03.83**

(54) **Oral compositions.**

(43) Date of publication of application:
**10.10.84 Bulletin 84/41**

(45) Publication of the grant of the patent:
**13.05.87 Bulletin 87/20**

(84) Designated Contracting States:
**AT BE CH DE FR GB IT LI NL SE**

(56) References cited:
**GB-A-1 290 627**

**JOURNAL OF THE AMERICAN CHEMICAL
SOCIETY, vol. 80, 5th June 1958, pages 2662-
2664 H.M. HAENDLER et al.: "The synthesis of
ammonium fluorometallates in methanol"**

(73) Proprietor: **UNILEVER PLC
Unilever House Blackfriars P.O. Box 68
London EC4P 4BQ (GB)**
(84) **GB**

(73) Proprietor: **UNILEVER NV
Burgemeester s'Jacobplein 1 P.O. Box 760
NL-3000 DK Rotterdam (NL)**
(84) **BE CH DE FR IT LI NL SE AT**

(72) Inventor: **Viccaro, John Peter
145-77 7th Avenue
Whitestone New York (US)**

(74) Representative: **Doucy, Robert Henry et al
Unilever PLC Patents Division P.O. Box 68
Unilever House
London EC4P 4BQ (GB)**

Courier Press, Leamington Spa, England.

## Description

This invention relates to compositions for the care of the oral cavity.

The efficacy of fluorine in the prevention of dental caries is well established (see for example US Patents 3 029 191, 3 070 510 and 3 227 617). The acceptance of fluoride ions as an anticariogenic agent is based essentially on the observation that among the many topical agents that have been tested in clinical trials only those that contain fluorine have been effective in reducing caries. Fluorine-free salts tested have not prevented caries formation.

It is also known that zinc salts inhibit the growth of dental plaque (see US Patents Nos. 4 022 880, 4 082 841, 4 100 269, 4 152 819 and 4 154 815).

The present invention relates to oral compositions comprising an active compound effective both as an anti-caries agent and as an anti-plaque agent.

According to the invention there is provided an oral composition having anti-caries and anti-plaque activity comprising 0.01 to 3% by weight of zinc ammonium fluoride ($ZnNH_4F_3$).

The synthesis of $ZnNH_4F_3$ is described in J.Am.Chem.Soc. 80, 2662, 1958.

In the preparation of therapeutic or prophylactic oral compositions incorporating $ZnNH_4F_3$, a suitable carrier or oral media well known in the art is used. Adjuvants such as colouring agents, flavours, humectants, abrasives, detergents, preservatives, emollients and the like and other therapeutic agents compatible with $ZnNH_4F_3$ may also be included.

The oral composition is preferably in the form of a toothpaste or mouthwash. A particularly suitable amount of the $ZnNH_4F_3$ is such as to provide from 1000 to 1500 ppm of fluoride ion. However, the amount may conveniently range from 100 to 3000 ppm.

The following Experiments and Examples illustrate the invention. Percentages are by weight.

The $ZnNH_4F_3$ employed in the following experiments was synthesised according to the method of Haendler and Johnson (J.Am.Chem.Sec. 80, 2662, 1958). The analytical details were as follows:

|  | Theoretical % | Analytical % |
|---|---|---|
| Zn | 46.58 | 46.90 |
| $NH_4$ | 12.83 | 12.26 |
| F | 40.59 | 40.96 |

Cariogenic Bacteria used for testing the effect of $ZnNH_4F_3$ were *Streptococcus mutans* (strains coded 6715 and OMZ-176), *Streptococcus sanguis* (strains coded 10556 and 10557), *Streptococcus salivarius* (strains coded SS4 and H257, and *Lactobacillus casei*. All of the above organisms were maintained as lyophilised cultures until the time for use.

An aqueous solution of $ZnNH_4F_3$ has a pH of 5.8. The preferred pH of oral compositions containing $ZnNH_4F_3$ is from 4.5 to 8.0. A 1000 ppm $F^-$ solution was stable. It did not become hazy on standing after several weeks. In contrast, a $SnF_2$ solution developed turbidity, probably due to oxy-fluoride formation, after 24 hours at room temperature.

At normal temperature and pressure $ZnNH_4F_3$ is more soluble (about 2.7%) than $ZnF_2$ (1.5%).

Solutions of $ZnNH_4F_3$ exhibited 100% availability of $F^-$ ion at 1000 and 2500 ppm F levels; the ionised fluoride was determined by the Orion-Selective Fluoride electrode (Model 96-09).

$ZnNH_4F_3$ was much less astringent and metallic tasting than the corresponding uncomplexed fluoride salts at equal molar concentrations. More particularly, $ZnNH_4F_3$ was considerably less astringent than the following (ranked in descending order of astringency): zinc phenolsulphonate, $ZnSO_4$ and $ZnF_2$ at 0.11M concentration which would be equivalent to approximately 6000 ppm $F^-$ levels. These results were the unanimous decisions of a blind study consisting of a tasting-panel of three judges.

$ZnNH_4F_3$ at 1000 ppm F levels had no effect on the transparency or taste of oral compositions.

It is generally agreed that oral bacteria are responsible for tooth decay. Therefore, a reduction of the cariogenic flora usually results in a decrease in caries formation. The inhibitory action of the test compounds was determined by adding each salt, at known concentrations, to a culture of cariogenic bacteria. After 24 h at 37°C bacterial growth was monitored as a change in optical density. A dose response curve (concentration of salt vs. growth) for each compound against each bacterial isolate was plotted in order to obtain the concentration of test agent which would cause a 50% inhibition in growth (ID/50) This is a useful ranking index since it is not always possible to obtain complete inhibition with weak germicidal agents. Only those compounds which were able to elicit an ID/50 response were considered as inhibitory agents.

The medium used for bacterial growth experiments was Trypticase Soy Broth (B.B.L.: Baltimore Biological Labs., Baltimore, MD) supplemented with 0.1% Tween 80 (the word Tween is a trade mark) and 0.05% sodium thioglycollate. The test organisms, as lyophilised cells, were grown overnight in 50 ml of growth medium in a vacuum desiccator with an atmosphere of 97% $CO_2$/3% $H_2$ at 35°C. These cultures were then used at a 0.2% inoculum to prepare second stage seed flasks. The test media (10 ml) consisting of growth medium plus the potential inhibitors, contained in 16×120 mm screw top test tubes, were inoculated with the second stage seed cultures to give an absorbancy reading of 0.05. Immediately after inoculation the cultures were incubated for 24 hours under anaerobic conditions.

The growth medium was sterilised by autoclaving, whereas the potential inhibitors (as aqueous solutions) were sterilised by filtering each solution separately through a Millipore membrane (0.22 μm).

Absorbance at 600 nm versus a medium control was used as a measure of growth on a Beckman Model B spectrophotometer. All absorbancy readings were determined in 16 mm×120 mm Kimax test tubes. This instrument was linear to 0.60 optical density units; thus, all cultures displaying values above this range were diluted with the control, accordingly.

### TABLE I
### Inhibition of bacterial growth

| | S. mutans | | S. salivarius | | S. sanguis | | L. casei |
|---|---|---|---|---|---|---|---|
| | | | | ID/50 (mM/L) | | | |
| Salts | 6715 | OMZ-176 | SS4 | H-257 | 10556 | 10557 | |
| ZnSO$_4$ | 5.74 | 7.50 | 6.00 | 0.75 | 0.36 | 0.40 | 10.45 |
| * ZnNH$_4$Cl$_3$ | 4.10 | | 6.50 | | 0.46 | | 10.00 |
| * ZnNH$_4$F$_3$ | 1.62 | 3.05 | 1.88 | 0.34 | 0.21 | 0.25 | 4.70 |
| * NaF | 7.3 | 16.30 | 13.40 | 3.77 | 1.18 | 1.82 | >12.00 |

The above values are the means of triplicate experiments. Due to a limited amount of ZnNH$_4$Cl$_3$, half the organisms were excluded from evaluation using this material.

The results show that ZnNH$_4$F$_3$ was more inhibitory than the non-fluoride salts and NaF, with no apparent changes in specificities. The ID/50 values, recalculated as ppm F⁻, again show (Table II) that ZnNH$_4$F$_3$ is more active than NaF.

### TABLE II
### Inhibition of bacterial growth by fluorine salts

| | S. mutans | | S. salivarius | | S. sanguis | | L. casei |
|---|---|---|---|---|---|---|---|
| | | | | ID/50 (ppm F) | | | |
| Salts | 6715 | OMZ-176 | SS4 | H-257 | 10556 | 10557 | |
| NaF | 138 | 309 | 255 | 72 | 22 | 35 | 352 |
| * ZnNH$_4$F$_3$ | 92 | 174 | 104 | 19 | 12 | 14 | 268 |

One of the mechanisms whereby fluoride decreases caries formation is that the fluoride reduces the acid solubility of dental enamel. This trait has been demonstrated repeatedly by *in vitro* dissolution tests. It is well established that the fluoride ion reacts with the hydroxyapatite of tooth enamel to form principally CaF$_2$ and fluoroapatite. The relative importance of the two products is still questionable but fluoroapatite is more desirable. Nevertheless, in either case the altered tooth's surface is less susceptible to demineralisation from acids produced by oral bacteria than the unmodified enamel. The cation of the fluoride salt can also react with dental enamel to yield the corresponding insoluble phosphates which in turn may reduce enamel solubility.

It was, therefore, a purpose of the following study to determine the efficacy of ZnNH$_4$F$_3$ in reducing hydroxyapatite solubility. The ammoniated salt was compared with SnF$_2$, NaF and Na$_2$FPO$_3$ using a dissolution system employing hydroxyapatite discs described by Forward (*Caries Research* 11:9-15, 1977). Demineralisation of a hydroxyapatite disc was determined by measuring the amount of phosphate released from a given area (123 mm$^2$) of the disc after a standard exposure time to a buffered solution of the test agent in the following manner. Hydroxyapatite discs of uniform size (13 mm diameter) were prepared by cold vacuum compression of a mixture of column chromatography hydroxyapatite powder (Bio-Gel HTP, Bio-Rad Labs, Richmond, CA) and polyethylene powder. Each disc was embedded in a block of paraffin (19 mm square) exposing only one face. The block was then mounted on a plastic rod which was placed in the chuck of a motor. The mounted discs were hydrated in distilled water for 1 min., avoiding air bubble formation on the disc's surface by slowly rotating the paraffin block. Upon removal, excess water

was removed from the disc with a paper tissue. The hydrated discs were subsequently submerged into the buffered test solution for 1 min., followed by a water rinse of 10 ml and a 1 min. immersion in 50 ml of distilled water. The disc was again blotted lightly with a tissue and at this point was ready for dissolution. The block-disc preparation was immersed (depth 7.6 cm) in 100 ml of 1 M acetate buffer, pH 4.65 contained in a plastic jar. Dissolution experiments were performed at 37°C by rotating the mounted discs at 350 rpm ±10 rpm for 30 min. After this time the extent of reaction of the test compound with the hydroxyapatite disc was determined by analysing the acetate buffer for phosphate using the method of Chen et al (Anal. Chem. 28:1756—1758, 1956). Percent reduction in dissolution was the difference in phosphate levels between a disc treated with buffered-test agents and a control disc treated with buffer alone.

Table III shows the relative demineralisation occurring when hydroxyapatite is exposed to buffered solutions of the representative fluorides. It can be seen that the degree of demineralisation caused by 1000 ppm fluoride solutions varies with each fluoride salt and the pH of the solution, but in every case dissolution was reduced, particularly at the lower pH values. This is consistent with past evidence that acidic conditions increase the rate of formation of $CaF_2$ and fluoroapatite. The results also indicate that the Sn and $ZnNH_4$ salts are similar in activity and both are considerably more effective in reducing demineralisation than NaF and $Na_2FPO_3$ at all pH values. These data indicate that the presence of $ZnNH_4$ cations enhance the anti-cariogenic effects of fluoride.

## TABLE III
### Dissolution of hydroxyapatite discs following fluoride treatment

| Fluoride solutions at 1000 ppm F⁻-Conc. | pH | % Reduction in dissolution |
|---|---|---|
| NaF | 4.0 | 44 |
| NaF | 5.0 | 36 |
| NaF | 6.0 | 31 |
| NaF | 7.0 | 31 |
| $Na_2FPO_3$ | 4.0 | 26 |
| $Na_3FPO_3$ | 5.0 | 23 |
| $Na_2FPO_3$ | 6.0 | 22 |
| $Na_2FPO_3$ | 7.0 | 20 |
| $SnF_2$ | 4.0 | 57 |
| $SnF_2$ | 5.0 | 54 |
| $SnF_2$ | 6.0 | 45 |
| $SnF_2$ | 7.0 | 43 |
| $ZnNH_4F_3$ | 4.0 | 52 |
| $ZnNH_4F_3$ | 5.0 | 49 |
| $ZnNH_4F_3$ | 6.0 | 43 |
| $ZnNH_4F_3$ | 7.0 | 40 |

The above values are means of triplicate experiments.

Dental plaque is a film on the surface of teeth. This layer is the result of bacterial growth and is composed chiefly of microorganisms, proteinaceous materials and microbial by-products, such as glucans and organic acids. Plaque is not only primarily responsible for caries formation but it is also implicated in

4

gingival diseases. Because the results described herein above have demonstrated that $ZnNH_4F_3$ possesses antibacterial activity, it is obvious that $ZnNH_4F_3$ will also reduce plaque formation.

The *in vitro* test used to compare the antiplaque effects of $ZnNH_4F_3$ vs $ZnSO_4$ is now described.

The *in vitro* plaque was initiated on uniformly sized aluminium plummets which were previously coated for 20 min. with wax stimulated whole saliva. These coated plummets were then placed in a Trypticase (B.B.L.) 1% sucrose growth medium inoculated with clinical plaque samples. After 5 h of incubation at 37°C the plummets were immersed in a solution containing equal volumes of whole saliva and the test compound for one minute and subsequently suspended overnight (37°C) in a 20% solution of saliva supernatant. On the second day the plummets were retreated with the test solution (1 min.) and reincubated in inoculated Trypticase-sucrose growth medium for 5 h. After this period of time the plaques were rinsed in distilled water for 5 min. The plaques were then removed from the plummets by sonication in 10 ml distilled water and quantitated in a Unicam SP 500 spectrophotometer at 570 nm.

A test compound showed antiplaque activity if the plaque mass (optical density reading) was less than the control plaques treated with water. Five replicas per test compound were examined.

Table IV clearly indicates that $ZnNH_4F_3$ is an effective antiplaque agent. $ZnSO_4$ was used as a standard soluble zinc salt and the results obtained are consistent with the bacterial theory of plaque formation.

TABLE IV
Antiplaque study

| Test solutions zinc conc. as mM/L | Percentage inhibition of growth of artificial plaque* | |
| --- | --- | --- |
| | $ZnSO_4$ | $ZnNH_4F_3$ |
| 6.0 | 47±14 | 45±14 |
| 15.0 | 51±14 | 48±14 |
| 30.0 | 64±14 | 60±14 |

*The values are means ±95% confidence limits.

The fluoride concentration from $ZnNH_4F_3$ at these test levels does not affect plaque formation (Skjorland, Gjermo & Rolla, Scand, J. Dent. Res. 86:103—117, 1978). There was no difference in activity between $ZnSO_4$ and $ZnNH_4F_3$ and this is consistent with the fact that the zinc is freely available due to the rather weak complexing between the Zn and $NH_4$ (log K 2.18—2.59).

An animal study was conducted to establish the effect of $ZnNH_4F_3$ on the incidence of caries. The animal study was conducted as follows. Osborne-Mendel rats were interbred in a closed colony on site and the animals mated randomly. The breeding animals were maintained on a mixed cereal (corn, wheat, soybean) fish and bone meal diet prior to mating. Rats were weaned generally between 22—24 days so that experiments could start at a predetermined date. The animals from one litter were randomly assigned one, or preferably two, to each of the various treatments and the total number of animals per treatment was sixteen. Each animal was weighed, numbered and caged separately in a stainless steel wire cage which restricts coprophagy. The animals were then maintained on a cariogenic diet (sugar 66%, skim milk 32%, liver powder 2%) and fed sterile distilled water. The test solution was applied topically with a fine sable haired brush to each rat's molar teeth for 15 sec. twice daily for the first 2 weeks and once daily for the third week. After 21 days the animals were sacrificed with chloroform and weighed. The two separate mandible and the complete upper jaw were then excised and the mandibles defleshed by means of a scalpel and dental chisel after immersion in neutralised formal saline for 48 h. The molar teeth were mounted, sectioned longitudinally, and stained with Schiff's reagent and scored by the method of *Konig et al, Dtsch. Zahn-, Mund.- and Kieferheilk* 29: 99-127 (1958). Using a 40X magnification the lesion in each fissure was rated for severity on a four point scale, of ATBC. Scoring lesion severity was as follows: (A) Pink colouration in enamel within the fissure; (T) Pink colouration at the enamel-dentine junction but without loss of dentinal material; (B) Colouration and loss of material at the enamel-dentine junction; (C) Loss of enamel material from within the fissure. At the conclusion of the experiment the number of lesions and severity score for each treatment group was averaged and the percent reduction in caries based on the water control was calculated.

The dental caries data are presented in Table V. Only the severe lesions (B&C) were scored in this experiment since the object of the study was to determine whether a significant difference in performance existed between fluoride compounds. Numerically all of the experimental groups demonstrated a reduction in caries which were statistically significant from the controls; however, there was no significant difference in activity between the fluoride salts. While the results achieved with $ZnNH_4F_3$ were not different from the reductions obtained with NaF and NaMFP (sodium monofluorophosphate, $Na_2PO_3F$), they nonetheless demonstrate that significant dental caries reduction can be achieved with $ZnNH_4F_3$.

TABLE V
Rat caries study

| Group No. | Treatment solution | $F^-$-ppm | Caries/rat* | % reduction |
|---|---|---|---|---|
| 1 | $F^-$ free water | — | 5.25 | — |
| 2 | NaF 0.22% | 1000 | 3.69 | 29.7 |
| 3 | $Na_2PO_3F$ 0.8% | 1000 | 3.69 | 29.7 |
| 4 | $ZnNH_4F_3$ 0.25% | 1000 | 3.63 | 30.9 |

*0.649—Critical difference in the means at 95% confidence level.

The following are examples of a toothpaste and a mouthwash composition of the invention.

Example 1

The following is an example of a toothpaste composition according to the invention—

| | |
|---|---|
| Silica xerogel | 13.00 |
| Silica aerogel | 22.00 |
| Sorbitol (70% solution) | 41.50 |
| $ZnNH_4F_3$ | 0.24 |
| Sodium lauryl sulphate | 7.00 |
| Colour | 0.04 |
| Flavour | 3.00 |
| Sodium saccharin | 0.12 |
| Sodium benzoate | 0.06 |
| Titanium dioxide | 0.35 |
| Glycerine | 7.53 |
| Water | Balance to 100% |

Example 2

The following is an example of a mouthwash composition according to the present invention.

| | |
|---|---|
| Ethanol | 15.00 |
| Propylene glycol | 10.00 |
| Glycerol | 12.00 |
| Flavour, colour | 0.90 |
| Polyoxyethylene (20) monolaurate | 2.10 |
| Sodium lauryl sulphate | 0.33 |
| $ZnNH_4F_3$ | 0.024 |
| Buffer | 18.00 |
| Hydrochloric acid (to pH 4.1) | q s |
| Water | Balance to 100% |

Although the examples herein show the use of $ZnNH_4F_3$ in dentifrice and mouthwash preparations, the compounds of the present invention may also be incorporated in other oral preparations or formulations such as for example chewing gums and lozenges.

**Claims**

1. An oral composition having anti-caries and anti-plaque activity comprising 0.01 to 3% by weight of zinc ammonium fluoride ($ZnNH_4F_3$).

2. An oral composition as claimed in Claim 1, wherein the amount of $ZnNH_4F_3$ is sufficient to provide 1000 to 1500 ppm of available fluoride ion.

3. An oral composition as claimed in Claim 1 or Claim 2 in the form of a toothpaste or mouthwash.

**Patentansprüche**

1. Ein orales Präparat mit Antikaries- und Antiplaque- Wirksamkeit, dadurch gekennzeichnet, dass es 0.01 bis 3 Gew.% Zinkammoniumfluorid ($ZnNH_4F_3$) enthält.

2. Ein orales Präparat wie in Anspruch 1 beansprucht, dadurch gekennzeichnet, dass der Gehalt an $ZnNH_4F_3$ ausreichend ist, um 1000 bis 1500 ppm verfügbares Fluoridon zur Verfügung zu stellen.

3. Ein orales Präparat wie in Anspruch 1 oder Anspruch 2 beansprucht in Form einer Zahnpaste oder eines Mundwassers.

**Revendications**

1. Composition buccale possédant une activité anti-caries et anti-plaque, qui comprend de 0,01 à 3% en poids d'un fluorure de zinc-ammonium ($ZnNH_4F_3$).

2. Composition buccale selon la revendication 1, dans laquelle la quantité de $ZnNH_4F_3$ est suffisante pour fournir 1000 à 1500 ppm d'ion fluorure disponible.

3. Composition buccale selon la revendication 1 ou 2, qui est sous forme d'une pâte dentifrice ou d'un bain de bouche.